# EUROPEAN PATENT APPLICATION

(11) **EP 1 444 967 A1**
(43) Date of publication of application: **11.08.2004**
(21) Application number: 03258233.0
(22) Date of filing: 30.12.2003
(51) Int. Cl.: A61F 2/08

(54) **Anterior cruciate ligament reconstruction system and method of assembling same**

(30) Priority: 04.02.2003 US 445259 P
(71) Applicant: Coapt Systems, Inc., Palo Alto, CA 94303 (US)
(72) Inventor: Elson, Robert J., Los Altos Hills, California 94022 (US); Jacobs, Daniel, Palo Alto, California 94306 (US); Magen, Hugh E., San Francisco, California 94110 (US)
(74) Representative: Jackson, David Spence

(57) **Abstract**

A soft tissue reconstruction system uses a method for fixating and anchoring a graft within a bone tunnel (72), and is especially adapted for reconstructing a defective anterior cruciate ligament. The reconstruction system (1) is assembled ex-vivo by threading a graft (14) through an opening disposed on a first shaft connected to a first bone anchor (18), and fixing a ring member (34) around the graft (14) and a second shaft (12) for securing the graft (14) to the second shaft (12) that is connected to a second bone anchor (36). After the assembled reconstruction system (1) is inserted into the bone tunnel (72), the first bone anchor (18) is anchored to bone material (70) adjacent one end of the bone tunnel, and the second bone anchor (36) is anchored to bone material (71) adjacent another end of the bone tunnel (72).

## Description

This application claims priority of U.S. Provisional Application No. 60/445,259, filed February 4, 2003.

### FIELD OF THE INVENTION

The present invention relates to the field of orthopedic surgery, and more particularly to an orthopedic surgical device or system (and the implementation thereof) that is used to reconstruct soft tissue, such as tendons and ligaments, within the knee or other parts of the body.

### BACKGROUND OF THE INVENTION

The present invention is primarily directed to the reconstruction of the anterior cruciate ligament (ACL) of the knee. The ACL is a two-bundle ligament that helps to stabilize the knee joint, and prevents posterior displacement of the femur on the tibia and hyperextension of the knee joint.

The ACL has poor healing properties, and thus, an untreated injury potentially leads to recurrent "giving-way" episodes, further damage to the menisci and articular cartilage, and possible progression to osteoarthritis (Brown et al., Clinics in Sports Medicine 18(1): 109-170 (1999)). Therefore, management of these injuries has evolved from nonoperative treatment through extracapsular augmentation and primary ligament repairs to the currently used open or arthroscopically assisted anterior cruciate ligament reconstruction. A complete understanding of the anatomy and biomechanics of the ACL has not been attained in the field of orthopedics, and thus, there is much active research in both normal and reconstructed knee biomechanics in order to develop improved systems for ACL reconstruction.

A typical surgical procedure for ligament replacement and reconstruction involves obtaining a tissue graft or a suitable synthetic graft to replace the damaged ligament. The graft may come from either another part of the patient's body (autograft), from a cadaver donor (allograft), or the graft may be synthetically manufactured. Current research may also lead to the use of grafts derived from animals (xenograft). In addition, the graft may itself be comprised entirely of soft ligament tissue or, alternatively, a combination of soft tissue attached to a "tendon bone block" on either end of the graft (a bone-tendon-bone graft). Methods for placement of such grafts are generally described in Goble et al., U.S. Pat. Nos. 4,772,286; 4,870,957; 4,927,421; 4,997,433; 5,129,902; 5,147,362; U.S. Pat. No. Re. 34,293; Kurland, U.S. Pat. No. 4,400,833; Jurgutis, U.S. Pat. No. 4,467,478; Hilal et al., U.S. Pat. No. 4,597,766; Seedhom et al., U.S. Pat. No. 4,668,233; Parr et al., U.S. Pat. No. 4,744,793; Van Kampen, U.S. Pat. No. 4,834,752; and Rosenberg, U.S. Pat. No. 5,139,520. Dore et al. teach the use of a tension spring for use as an artificial prosthetic ligament (U.S. Pat. No. 4,301,551).

Although the use of a bone-tendon-bone graft may provide the advantage of effective healing due to the efficient biointegration of the bone graft to the bone host, the harvesting of a bone-tendon-bone graft typically results in extensive morbidity to the donor knee joint, thus lengthening the patient's resumption of normal physical activity. It is, therefore, often preferable to harvest grafts made up entirely of soft tissue, e.g., a hamstring tendon, because such a procedure involves less donor site morbidity. On the other hand, it has historically been more difficult to effectuate and maintain accurate fixation of such grafts throughout the healing period where high-tension forces of the knee may act to disrupt the graft construct (e.g. via fixation device slippage or graft failure).

When performing ACL reconstruction with a soft tissue graft, the selected material is attached (fixated) to natural insertion sites of the patient's damaged ligament. Many devices and procedures used for orthopedic ligament reconstruction are specifically designed both to overcome the myriad of difficulties for fixating soft tissue ligament grafts to the hard tissue bone surface, and for enabling the patient to return to a full range of activity in as short a period of time as possible. To that end, medical researchers have attempted to duplicate the relative parameters of strength and flexibility found in natural ligaments of the body. Unfortunately, many existing procedures have proven inadequate for immediately restoring adequate strength and stability to the involved joint. Furthermore, even if immediate achievement of knee stability is achieved, many current methods are ineffective at maintaining such stability throughout the period when the mechanical phase of graft fixation is ultimately superceded by a permanent biological phase of graft integration.

Conventional ACL reconstruction procedures typically include the formation of a tunnel through the patient's femur and tibia bones in the knee joint, and implanting an organic or synthetic ligament in the bone tunnel that eventually attaches itself to the bone and to hold those two bones together. One difficulty in effectively implanting a fully effective ligament reconstruction is the surgeon's need to balance a number of variables leading to "trade-offs". Such variables include the need to position a sizable graft ligament at a precise location within the joint while minimizing trauma to the host bones, and while constrained by the need to use the smallest possible bone tunnel. When creating the ligament reconstruction, it is generally important to use as large a graft ligament as possible, to (i) provide high graft strength along the length of the graft to prevent subsequent rupture, and (ii) provide an extensive supply of collagen material to facilitate effective integration of the graft ligament into the bone. At the same time, the physics of the knee joint dictate the location of the graft fixation points and hence the location of the bone tunnel. Of course, the particulars of the surrounding anatomy may affect graft ligament size and/or bone tunnel size.

Another important consideration for ACL reconstruction is the ability to achieve a desirable final resting tension on the graft, which is important for attaining a desirable joint stability after healing. Many ACL reconstruction systems and techniques allow the tension to be set during insertion of the graft, but not subsequent to tissue fixation and bone anchoring, and especially not subsequent to the knee being subjected to its range of motion. Thus, the final intra-operative resting tension on the graft ligament is either unknown or unadjustable. Ideally, the graft ligament should be tight enough to provide stability to the joint rather than being simply a "checkrein" incurring a load only at the extremes of knee motion. If it is determined after tissue fixation and bone anchoring (and possibly after the knee is moved through its range of motion) that the desired ligament tension was not achieved, most ACL reconstruction systems and techniques offer little or no corrective options. Moreover, anchor structures, such as those in Johnson (U.S. Pat. No. 5,562,668), are complex, bulky, and difficult to use properly. Methodologies for "pretensioning" the graft prior to fixation are shown in Daniel et al. ('542) and in Goble et al. (U.S. Pat. Nos. 5,037,426; 5,713,897).

Another variable to be addressed with ACL reconstruction involves the balance between selecting appropriate bone anchoring locations for the reconstruction device, and selecting appropriate fixation of the soft tissue so as to approximate it to bony surfaces for healing. Conventional procedures may be separated into two general categories: 1) those that permit anchoring of the device within the bone tunnel (interior anchoring), and 2) those that utilize anchoring outside of the bone tunnel (external anchoring). External anchoring provides an advantage in that a substantial portion of the load on the graft may be borne by the stronger bone exterior or cortex. However, such external anchoring presents several problems. For example, external anchoring requires a longer graft to be harvested in order to reach the external fixation point. The presence of a longer segment of stretchable graft within the bone tunnel can have the "bungee cord effect" that can widen the tunnel, impede healing, and damage the graft. Also, the lack of immobilization of the graft at the articular orifice can lead to lateral motion (windshield or sundial effect), widening of the orifice, impeded healing, and damage to the graft. Anchoring the graft within the bone tunnel can overcome the problems of external anchoring, but can diminish the strength of the graft anchor since the bone interior is softer and provides an inferior anchoring point. Internal anchoring typically requires the use of devices that are destructive of the soft graft tissue (as described below). Finally, anchoring the ligaments entirely within the bone tunnel precludes the surgeon from properly adjusting the tension on the graft after it has been placed within the tunnel.

Devices that are currently used for anchoring grafts include pins, screws, baffles, bone blocks, staples, and washers. The use of "cross-pinning" (i.e., in which a pin, screw, or rod is driven into the bone transversely to the bone tunnel intersecting and "cross-pinning" a bone-tendon-bone in the bone tunnel or providing a ledge over which the soft tissue graft can be looped) to secure a graft is generally utilized for securing bone-tendon-bone grafts and soft tissue grafts.

As described above, a well-established method of maintaining a replacement graft at an anchor site entails the retention of the graft within the bone tunnel by an endosteal fixation device, such as an interference screw, to press at least one end of the graft against the interior wall of a bone space (see Mahony, U.S. Pat. No. 5,062,843; Roger et al., U.S. Pat. No. 5,383,878; Steininger et al., U.S. Pat. No. 5,425,767; Huebner, U.S. Pat. No. 5,454,811; Laboureau, EU 0 317 406). Grafts may be anchored between two elements, the inner one being deformable (U.S. Pat. No. 5,108,431), and they may be passed through a center of a device, creating tension by relative movement of elements (see DeSatnick, U.S. Pat. No. 5,571,184). However, such devices may create a gap between the bone and the ligament graft, thereby precluding maximal graft-tunnel contact at the point of immobilization, thus possibly impeding healing.

Interference screws, by definition, function by creating a tight fit between the graft and the surrounding bone. Such constructs require a continuous high-pressure load against both the graft and the surrounding bone, thus possibly leading to damage to the graft and erosion of the bone. Puncturing, piercing, and possible tearing of the graft is even more likely due to the additive loads present during flexion or extension of the knee or during high stress activities. Impeded healing or loosening of the interference fixation, and thus loss of fixation and graft slippage, can often result. Such an outcome could represent a failure of the operative procedure. Lastly, healing can be impeded because there is no separation between the fixation and healing portions of the graft. Tissue necrosis at the tissue fixation portion of the graft can impede healing to the adjacent bone.

As mentioned above, other procedures allow a surgeon to anchor the graft outside of the bone tunnel and to the external bone surface. These procedures, however, typically require the surgeon to use a graft having a length such that it extends beyond the cortex of the bone tunnel, and bends at approximately a 90 degree angle so that the graft end is flush against the external bone surface for securing to the external bone, which is not ideal. Stainless steel staples, buttons with sutures, and other related fixation devices have each been used for external anchoring, with limited success, because external fixation devices can have a high profile, are uncomfortable for the patient during healing, and can require a second surgery to remove them.

There is a need for a soft ligament tissue reconstruction system that separates bone anchoring, tissue fixation, and tissue healing from each other. Such a system needs to adequately present the graft tissue to adjacent soft bone for healing without necrosis. Lastly, such a system should allow for ex vivo assembly, where tissue fixation and system assembly can be more conveniently and accurately performed, yet provide in-situ adjustability to the graft tension (after bone anchoring, tissue fixation, and possibly even post-operation).

### SUMMARY OF THE INVENTION

The present invention solves the aforementioned problems by providing a method and apparatus for fixating and anchoring a graft within a bone tunnel. The apparatus can be completely assembled ex-vivo, and provides for graft tension adjustment even after full implementation.

One aspect of the present invention is a reconstruction system for inserting a graft into a pre-bored bone tunnel, which includes a first bone anchor for anchoring to bone material adjacent the bone tunnel, a shaft connected to the first bone anchor, and a ring member extending around the shaft and the graft and exerting a fixation force on the graft toward the shaft to secure the graft to the shaft, wherein the ring member is one of a ring compressed around the graft and the shaft, an elongated member wrapped around the graft and the shaft, and a ring excite compressed around the shaft and the graft.

Another aspect of the present invention is a method of assembling a graft reconstruction system by threading a graft through an opening disposed on a first shaft, wherein a first bone anchor is connected to the first shaft, and fixing a ring member around the graft and a second shaft such that the ring member exerts a fixation force on the graft toward the shaft to secure the graft to the second shaft, wherein the second shaft is connected to a second bone anchor, wherein the fixing of the ring member includes compressing the ring member around the graft and a shaft, or wrapping an elongated member around the graft and the shaft.

Other objects and features of the present invention will become apparent by a review of the specification, claims and appended figures.

### BRIEF DESCRIPTION OF THE DRAWINGS

Fig. 1 is a side view of the reconstruction system of the present invention.
Fig. 2 is a perspective view of the saddle member of the present invention
Fig. 3 is an exploded side view of the hook and cap members of the present invention.
Figs. 4A and 4B are side views of the femoral assembly of the present invention.
Fig. 5A is a side view of the tissue fixation and anchor bolt of the present invention.
Fig. 5B is a side view of the tissue fixation and anchor bolt of the present invention, with a unitary head and flange unit adjustably connected to the bolt shaft.
Fig. 6 is a perspective view of the tissue fixation ring of the present invention.
Fig. 7A is a side view of the bone anchor member of the present invention.
Fig. 7B is a cross sectional side view of the bone anchor member of the present invention implemented in a tibial bone tunnel.
Fig. 8 is a perspective view of the anchoring nut of the present invention.
Figs. 9A and 9B are top views of the compression band and heating element of the present invention, in different compression states.
Figs. 9C and 9D are top views of alternate embodiments of the compression band of the present invention.
Fig. 10 is a side view of the reconstruction system of the present invention just before insertion in the bone tunnel of the patient's knee.
Fig. 11A is a perspective view of an adjustment tool of the present invention.
Fig. 11B is an exploded view of the adjustment tool of the present invention.
Fig. 12A is a perspective view of the adjustment tool engaged with the tibial assembly bolt of the present invention.
Fig. 12B is a perspective view of the adjustment tool engaged with the tibial assembly bolt and nut of the present invention.
Fig. 13 is a side cross sectional view of the reconstruction system of the present invention anchored in the bone tunnel of the patient's knee.
Fig. 14 is a side view of a first alternate embodiment of the reconstruction system of the present invention.
Fig. 15A is a perspective view of the femoral assembly for the first alternate embodiment of the present invention.
Figs. 15B and 15C are side views of the femoral assembly for the first alternate embodiment of the present invention.
Fig. 15D is a perspective view of the femoral assembly for the first alternate embodiment of the present invention, with no clamp member.
Fig. 16 is a side view of the tibial assembly for the first alternate embodiment of the present invention.
Figs. 17A and 17B are side views of the femoral assembly for the first alternate embodiment of the present invention, illustrating how the sutures are threaded therethrough.
Fig. 18 is a side cross sectional view of the first alternate embodiment of the reconstruction system of the present invention anchored in the bone tunnel of the patient's knee.
Fig. 19 is a side view of the femoral assembly of the present invention, illustrating how the sutures can be threaded therethrough.

### DETAILED DESCRIPTION OF THE PREFERRED EMBODIMENTS

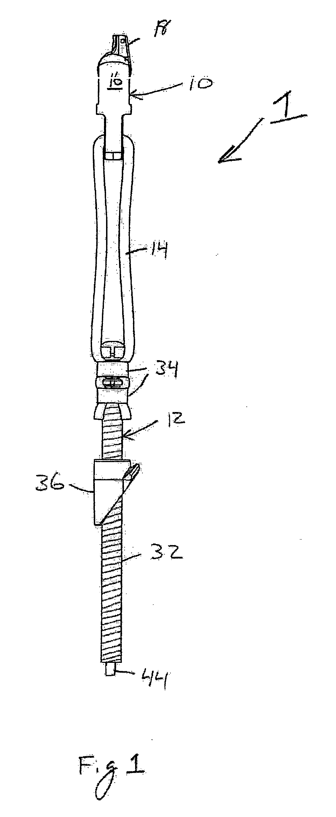

The present invention is a soft tissue reconstruction system 1, which is illustrated in its assembled form in Fig. 1. The reconstruction system 1 includes a femoral assembly 10 and a tibial assembly 12, with a graft 14 spanning therebetween.

Graft 14 as used herein includes any type of organic or inorganic, synthetic or natural, connective or muscular tissue, and/or any combinations thereof. Graft 14 may be autologous, allogeneic, xenogeneic, artificially engineered, or include mixtures thereof, with or without any preexisting bone attachments. Graft 14 can be a single strand of such material(s), or can be a plurality of strands of such material(s). One specific example generally includes any tissue and/or synthetic material suitable for anterior cruciate ligament (ACL) reconstruction. For instance, suitable ligament xenografts are described in U.S. Pat. No. 6,110,206 to Stone, and tissue-engineered tendons and ligaments are disclosed in U.S. Pat. No. 6,023,727 to Vacanti et al.

### Reconstruction System

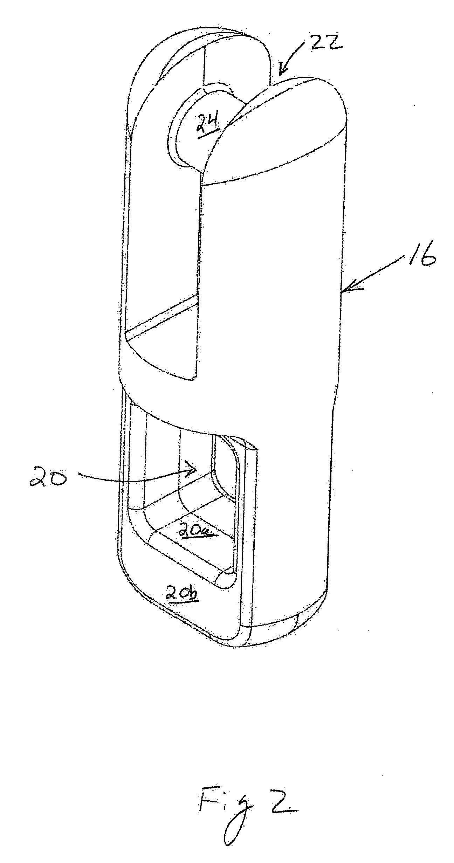

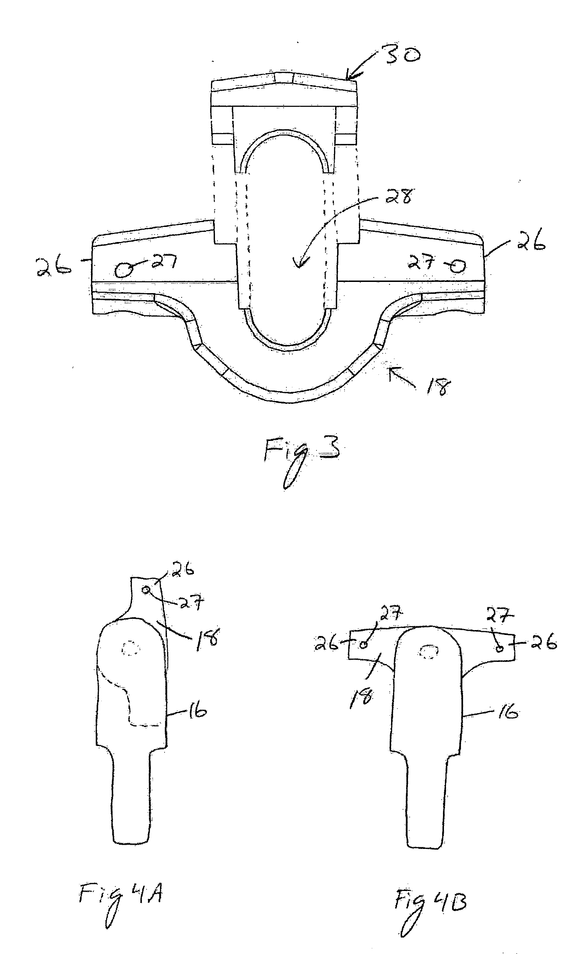

Femur assembly 10 includes a generally cylindrically shaped saddle member 16, and a hook member 18 (bone anchor), as best illustrated in Figs. 2 and 3. The saddle member 16 includes an opening 20 at one end (through which graft 14 can be looped or threaded), and a slot 22 at the other end. A tissue fixation surface 20a is defined in opening 20, and a tissue presentation surface 20b is defined laterally and below opening 20. A pin 24 extends across the slot 22, and is preferably but not necessarily integrally formed with saddle member 16 for strength. Saddle member 16 can be formed as a single unit, or in multiple pieces that snap together. Hook member 18 includes a pair of tabs 26 and a central opening 28 with a rounded bottom surface. Guide holes 27 are formed in tab members 26. The hook member 18 is rotatably (pivotally) attached to the saddle member 16 by passing one of the tabs 26 through the saddle's slot 22 so that pin 24 engages with the rounded surface of the hook member's central opening 28. A cap member 30 preferably having a bottom rounded surface is placed over the pin 24 and attached to the hook member 18 (e.g. with adhesive, ultrasonic welding, etc.). Once assembled, the hook member 18 can freely rotate about pin 24 between an insertion position as illustrated in Fig. 4A (where hook member 18 extends generally parallel to the saddle member 16) and an anchor position as illustrated in Fig. 4B (where hook member 18 extends laterally from saddle member 16). When in the insertion position, tabs 26 are contained within the width of the saddle member 16, which is ideally dimensioned to fit through a bone tunnel as described below. When in the anchoring position, tabs 26 extend laterally from saddle member 16 to increase the lateral dimensions of the femur assembly 10 for bone anchoring after it is passed through the bone tunnel. Cap member 30 is preferably not load bearing, but does prevent hook member 18 from disengaging from pin 24 and from bending under forces exerted onto the wings 26.

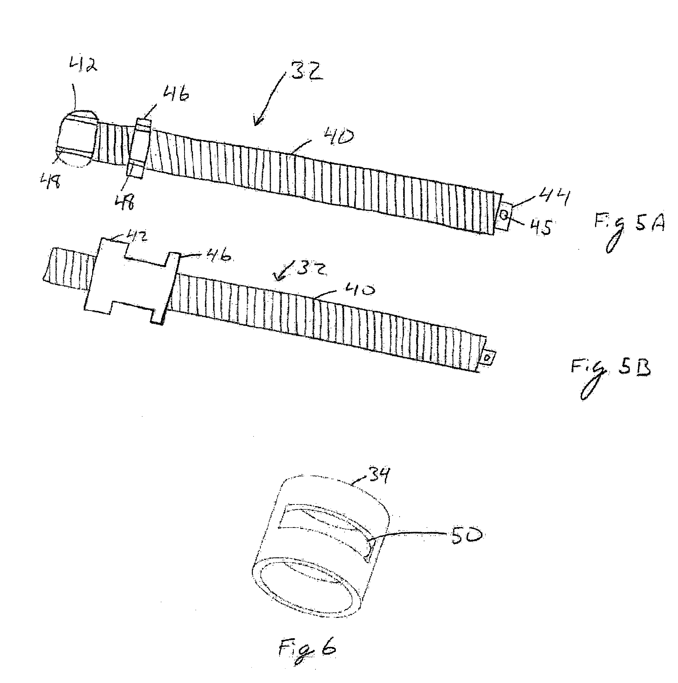

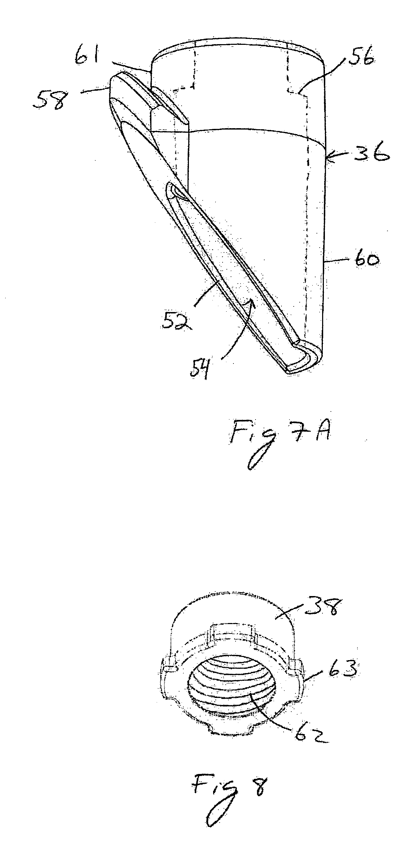

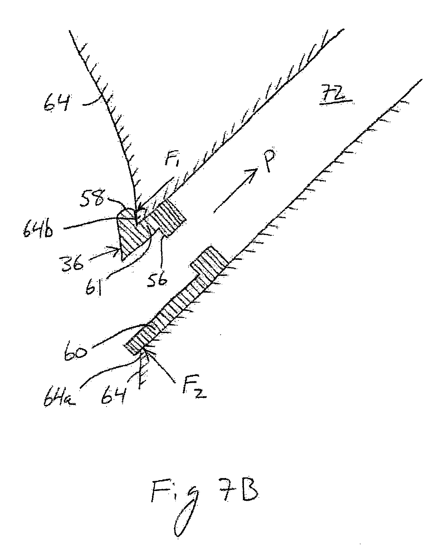

Tibial assembly 12 includes a bolt 32, a graft fixation ring 34, a bone anchor member 36 and a threaded nut 38. The bolt 32 is best illustrated in Fig. 5A, and includes a threaded shaft 40 having a bolt head 42 at one end, a tab 44 with a hole 45 formed therein extending from the other end, and a flange 46 disposed along the threaded shaft 40 adjacent to but spaced from bolt head 42. Flange 46 is preferably integrally formed with bolt 32, but instead may be threaded or otherwise attached (e.g. glued) onto bolt shaft 40 in a fixed or adjustable manner. Alternately, bolt head 42 and flange 46 could be integrally formed as a single unit that together is adjustably connected (e.g. with internal threads) to shaft 40 to adjust a location thereof along shaft 40 (and a distance between the single unit and bone anchor member 36) while preserving the distance between head 42 and flange 46, as illustrated in Fig. 5B. Both bolt head 42 and flange 46 include graft guide tabs 48 extending therefrom. Graft fixation ring 34 is best illustrated in Fig. 6, and is preferably a unitary hollow ring member with a pair of side apertures 50 sized to engage with flange 46. Bone anchor member 36 is illustrated in Fig. 7A, and is generally cylindrical in shape with a sloped surface 52 at one end, a bore 54 extending therethrough, an engagement protrusion or shoulder 56 in bore 54, and a tibial engagement projection 58 outwardly extending from the outer surface of the bone anchor member 36. The angle of sloped surface 52 is illustrated as around 55 degrees, but can be any angle that approximates the angle of the bone tunnel relative to the exterior surface of the bone into which it is formed. Sloped surface 52 allows bone anchor member 36 to be installed nearly flush against the surface of the cortical bone as well as providing other advantages, as described further below. Due to the sloped surface 52, the bone anchor member has one side wall 60 that is longer than an opposing sidewall 61. The tibial engagement projection preferably extends from the shorter sidewall 61.

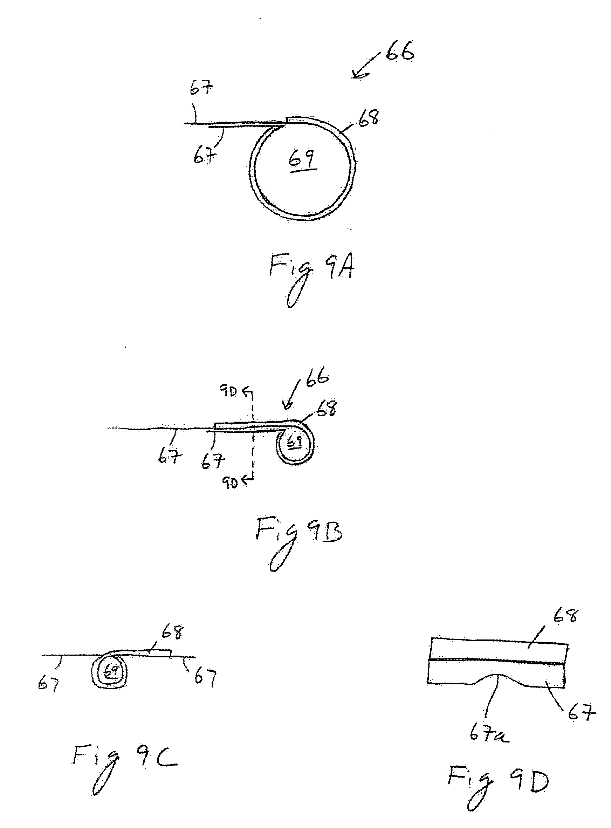

Threaded nut 38 is illustrated in Fig. 8, and has internal threads 62 for engaging with the threaded shaft 40 of bolt 32, and external tabs 63 that can be grasped for rotating nut 38 on bolt 32. Nut 38 is dimensioned to fit inside bore 54 and engage with engagement shoulder 56 (to secure bone anchor member 36 along bolt 32). External tabs 63 preferably have a height that is less than the height of the nut 38, which has been found to increase the stability of nut 38 when inside bore 54 of bone anchor member 36. External tabs 63 preferably engage with the sidewall portions of bore 54, to prevent the loosening of nut 38 after installation, to prevent the rocking of bone anchor member 36 relative to bolt shaft 40, and to provide support for the cylindrical sidewalls of bore 54.

While additional tibial engagement projections 58 could be added to the bone anchor member 36, a single such projection as shown is preferred. With a single projection 58 positioned on the shorter sidewall portion 61 and opposite the longer sidewall portion 60, and with the bone anchor member 36 having a sidewall outer diameter substantially equal to or slightly less than the inner diameter of bone tunnel 72 formed in the tibia/femur, the bone anchor member 36 is a self-centering and self-seating device, as shown in FIG. 7B. When tensile loading P along the bone tunnel 72 is presented (i.e. by tension in graft 14), bone anchor member 36 is configured to automatically seek the lowest energy state in providing a stable platform for graft fixation. More specifically, as bone anchor member 36 is pulled against tibial cortex 64, bone anchor member 36 inserts into bone tunnel 72 until tibia engagement projection 58 engages the tibial cortex 64 for bone anchoring. As the graft is then tensioned by tensile load P, projection 58 provides a longitudinal reactionary load to force F₁ at the tibia cortex 64. In addition, the long side wall 60 provides a lateral reaction load to force F₂ exerted by the tunnel sidewall to counter the moment generated between force F₁ and tensile load P, which stabilizes the bone anchor member 36 against the tibial cortex 64 and the walls of bone tunnel 72 formed therethrough. The cylindrical shape of the bone anchor member sidewall portions provides the necessary structural strength to counter force F₂ without any bending or failure thereof. Thus, the entire force that counters the tensile load P and prevents any longitudinal sliding along bone tunnel 72 is distributed mainly between two contact areas or regions 64a and 64b of the tibial cortex 64, which results in stable anchoring and adaptation to bone shape variances among different patients. Moreover, the sloped surface 52 and the position of projection 58 on the short sidewall 61 result in the bone anchor member 36 having a low profile that remains relatively flush against the bone surface without protruding therefrom by a significant distance.

Notwithstanding the above, a plurality of tibial projections 58 could be used, or even a continuous annular ring extending from the bone anchor member, where additional stability can be attained by excited compression (as described below) of the projection(s) or annular ring down onto the patient's bone so that a custom and secure fit is achieved.

The components of the femoral and tibial assemblies 10/12 may be made of various biocompatible metallic components, e.g., stainless steel, titanium, nickel-titanium alloys, etc, one or more compatible polymers, or biodegradable polymers synthesized from monomers comprising esters, anhydrides, orthoesters, and amides. Specific examples of biodegradable polymers include polyglycolide, polylactide, poly-alpha-caprolactone, polydioxanone, polyglyconate, copolymers of polylactide and polyglycolide, and the block and random copolymers of these polymers. Copolymers of glycolic, lactic, and other a-hydroxy acids may also be used. Porous materials and/or composites of absorbable polymers and ceramics, e.g., hydroxyapetite, are also suitable for use. Although the system components may comprise a single polymer or copolymer, generally for ease of construction by molding, the present invention is not so limited. For example, different system components may be made of different materials and/or material compositions. While the material(s) must be biocompatible, they also may be biodegradable, osteoconductive, and/or osteoinductive. Such "bio-integrated" materials are chosen and designed to cooperate in promoting optimal anchoring, fixation, and healing of the graft.

The present invention has been reduced to practice by making most of the femoral/tibial assembly components with a material composition of about 82% polylactic acid (PLA) and about 18% polyglycolic acid (PLGA). The PLA component gives the material strength, and the PLGA component gives the material its desired degradation properties. The graft fixation ring 34 has been made with a material composition of about 70% PLA and about 30% of poly DL-lactide, which produces the desired expansion, compression, fixation and degradation properties. It is expected that these percentage values may vary, sometimes significantly, to produce the desired performance.

### Reconstruction System Assembly

The assembly of reconstruction system 1 is performed ex-vivo in the following manner. The assembly is preferably begun after the overall length of the femoral/tibial bone tunnel 72 in the patient's knee is measured (e.g. by inserting a calibrated depth probe within the bone tunnel to measure its overall length). The formation of the bone tunnel through a patient's femur and tibia is well known, where the bone tunnel 72 includes a femoral portion 72a (through the femur) and a tibial portion 72b (through the tibia). Graft 14 is threaded (looped) through opening 20 of saddle member 16. Typically, graft 14 will include two graft strands, resulting in a double loop graft with four loose ends. The loose ends of graft 14 are then inserted through fixation ring 34 (which is preferably in its expanded state as described in detail below), and placed over bolt head 42 and bolt flange 46. Graft 14 is preferably held in place under tension (so that all graft strands will end up generally carrying the same load), where graft guide tabs 48 help evenly position the graft 14 around the bolt head/flange 42/46. The position of bolt head 42 and flange 46 along graft 14 is then set so that the overall length of the reconstruction system 1 matches the measured length of the patient's bone tunnel 72, such that the graft healing zones (discussed below) are optimally located within the bone tunnel. This is best accomplished by positioning the bolt head 42 along the graft 14 such that the distance from the hook member 18 to the bolt head 42 slightly exceeds the length of the femoral portion 72a of bone tunnel 72 plus the intra-articular length between the femoral and tibial bone tunnel portions 72a/72b (whereby the final length of the reconstruction system 1 is later set during insertion and final positioning of bone anchor member 36 along bolt shaft 40). The graft fixation ring 34 is then slipped over the bolt head 42 and bolt shaft 40 until apertures 50 of ring 34 engage with flange 46 (with flange 46 holding ring 34 in its desired position). Fixation ring 34 is then excite compressed_down onto graft 14 to secure graft 14 to bolt 32 and flange 46 (as further detailed below). Bone anchor member 36 is slid onto bolt shaft 40, and nut 38 is threaded onto shaft 40 until it is positioned to engage with shoulder 56 and prevents bone anchor member 36 from sliding past a desired bone tunnel insertion position along bolt shaft 40. The resulting assembled system is shown in Fig. 1.

It is important to ensure that the graft fixation ring 34 exerts enough fixation force against the graft 14 such that it will not siip relative to bolt 42 at anytime during the patient's recovery. It has been discovered that an ideal technique for securing graft fixation ring 34 involves "excited compression", where graft fixation ring 34 is "excite compressed" around the graft 14 and bolt 32. Excited compression of the ring 34 means mechanically compressing ring 34 while the ring material is in an excited state (where the molecules of the ring material have been sped up). Created the excited state can be achieved by, for example, subjecting the ring material to heat (e.g. via conduction), ultrasonic waves, radiation (e.g. visible, ultraviolet, and/or infrared light from a laser, RF, etc.) and so on. Once the excitation source and mechanical compressive force have been removed, the ring material exerts an inward force on the graft 14 that secures it to the bolt 32 in a very strong and reliable manner. It has also been discovered that "excited expansion" of the fixation ring 34 (i.e. mechanical expansion of the ring while in an excited state) before the ring is excite compressed can yield improved performance. Thus, while not necessary for many applications, excited expansion before excited compression may be preferred.

One example of excite expansion and excite compression of fixation ring 34 is heat expansion and heat compression via contact conduction, in the following manner. Graft fixation ring 34 is initially formed with an inner diameter that approximates its final compressed state. Ring 34 is then expanded by heating the ring and exerting out outward force on its inner diameter, so that the material expands until its inner diameter is significantly greater than its original size (e.g. as much as three times or more). The expansion force is removed after the material is cooled, so that the ring 34 maintains its expanded state. At this point, the ring is large enough to slide over graft 14, bolt head 42 and flange 46. After the graft 14 is properly positioned relative to bolt 32, the ring is then heated again, whereby the ring 34 tends to relax down toward its smaller (original) inner diameter. However, this relaxation does not produce enough force onto graft 14 to properly secure it in place on bolt 32. Thus, the ring 34 is mechanically compressed by an inward force while in its heated state. In this case, the mechanical compressive force is applied generally concentrically about bolt 32, so that the ring material is forced back down to a small enough inner diameter so that after cooling, a sufficient inward force is maintained on graft 14 by ring 34, thereby fixing graft 14 to bolt 32.

Fixation after excite compression is aided by the non-linear path created by flange 46, where graft 14 extending along bolt 32 has to bend up and over flange 46. Fixation is also aided by the threads on bolt shaft 40, which provide surface features that help prevent the graft 14 from sliding along bolt shaft 40. Additional or alternate gripping surface features could be added to the fixation portions of bolt shaft 40 (e.g. spikes, tines, etc.) to aid in fixation of graft 14 against bolt shaft 40. Such gripping surface features can also be added to the inner circumference of fixation ring 34, so long as such surface features can survive the ring expansion and compression. It should be noted, however, that the threads or other surface features on bolt 32 need not necessarily be present on the shaft's fixation portion, and that one or more portions of shaft 40 could be smooth.

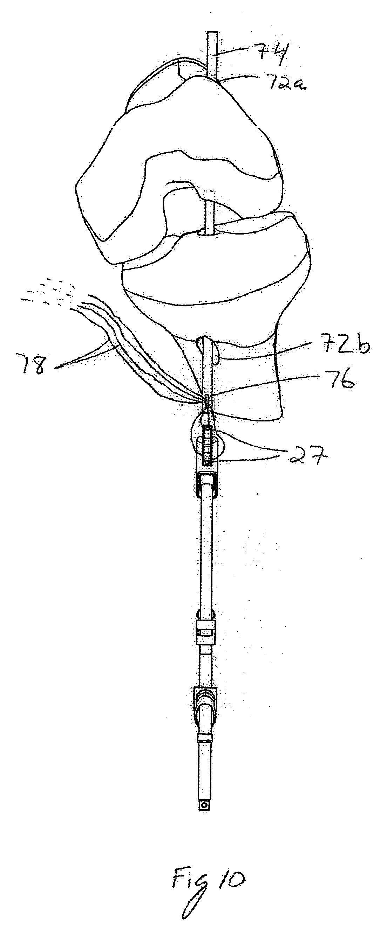

For optimal heat compression performance and a uniform fixture of graft 14 on bolt 32, the externally applied heat and inwardly concentric force are preferably applied evenly to ring 34 while ring 34 shrinks in size, and without applying excessive amounts of heat to the graft 14. This can be accomplished by utilizing a collapsing heat coil 66 that has been developed to more evenly heat and compress the graft fixation ring 34 without damaging the adjacent graft 14. The heat coil 66 is illustrated in Figs. 9A and 9B, and includes a flexible band 67 and a heating element 68 attached thereto. The band 67 is made of thermally conductive (and preferably biocompatible) material, such as stainless steel. The heating element 68 can be any conventional heat source (e.g. electrical coil heater, silk screened resistive traces, etc.) that heats band 67 preferably in a generally even manner. A thermally tolerant adhesive can be used to attach coil heater 68 to band 67. Alternately, coil heater 68 can be integrally formed with band 67.

The ends of band 67 are passed over each other so band 67 defines a compression aperture 69 (in which ring 34 is placed). By manually or mechanically manipulating the ends of band 67, the size of the compression aperture 69 is reduced (as shown in Fig. 9B relative to Fig. 9A), with the desired inwardly concentric force and thermal heat being evenly applied by band 67. The band 67 creates a circular heating surface that maintains a constant and continuous thermal, and force applying, contact with the ring 34 as the ring 34 is compressed in size. Once the ring 34 is cooled and the heating coil is removed, the ring 34 maintains the desired fixation force on graft 14 against bolt 32. In Figs. 9A/9B, the ends of band 67 are on the same side of compression aperture 69, where one end is pushed while the other is pulled to reduce the aperture size. Alternately, band 67 can be oriented so that both ends thereof can be pulled from opposite sides of aperture 69, as illustrated in Figs. 9C. In this case, the band 67 could include an aperture or slot through which the band can loop through so that the band 67 remains concentrically centered over ring 34. Also, band 67 may include a channel 67a formed in its heating/compression surface as illustrated in Fig. 9D, to accommodate flange 46 and ensure the compressive force is directed primarily on ring 34.

The ideal elevated temperature(s) associated with the excited state(s) used to expand and then later compress the graft fixation ring 34 will vary based upon its composition. If the temperature is too low, ring 34 may crack upon expansion or compression. If the temperature is too high, then the material forming ring 34 will become too soft and tend to flow in a liquid like manner. During compression, the ring material needs to be stiff enough to drive the tendon against bolt 32 (and flange 46 thereon), yet be soft enough to compress down in size. For the PLA and poly DL-lactide composition described above, expansion and compression temperatures of around 55-60°C were successfully used, using a linear pulling force of around 180 lbs on the ends of band 67. In order to minimize the risk of graft damage, the heating and compression of ring 34 is performed as quickly as possible (e.g. preferably less than 1 minute).

It should be noted that techniques other than using an excite compressed ring member for forming graft fixation ring 34 are within the scope of the present invention. For example, graft fixation ring 34 could be a metal ring crimped or compressed around graft 14 to provide the desired sustained inward fixation force. Or, graft fixation ring 34 could be an elongated member (such as a wire, a suture or even a well known tie-wrap device with locking member) wrapped around the graft 14 under sufficient tension to create the desired inward fixation force.

### Reconstruction System Implementation

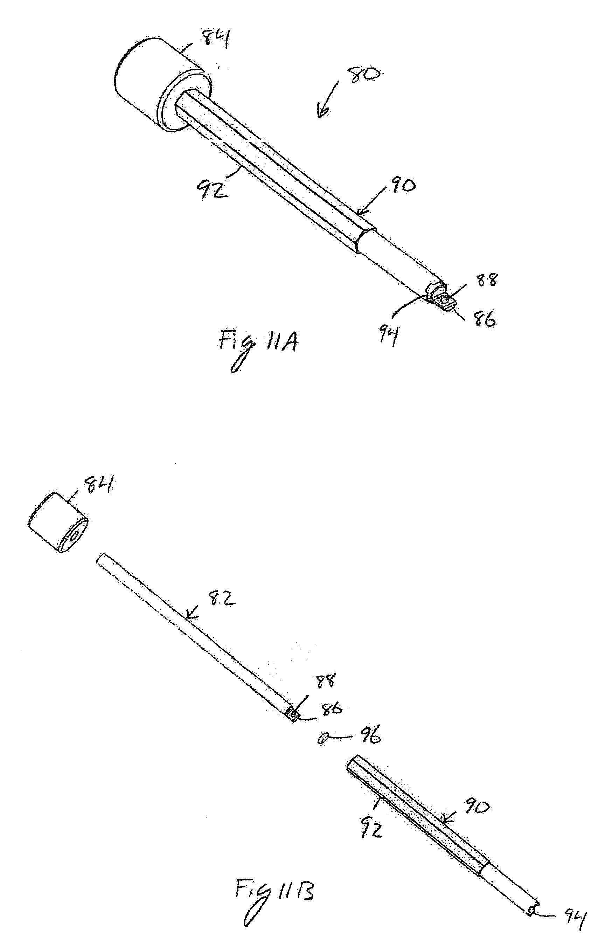

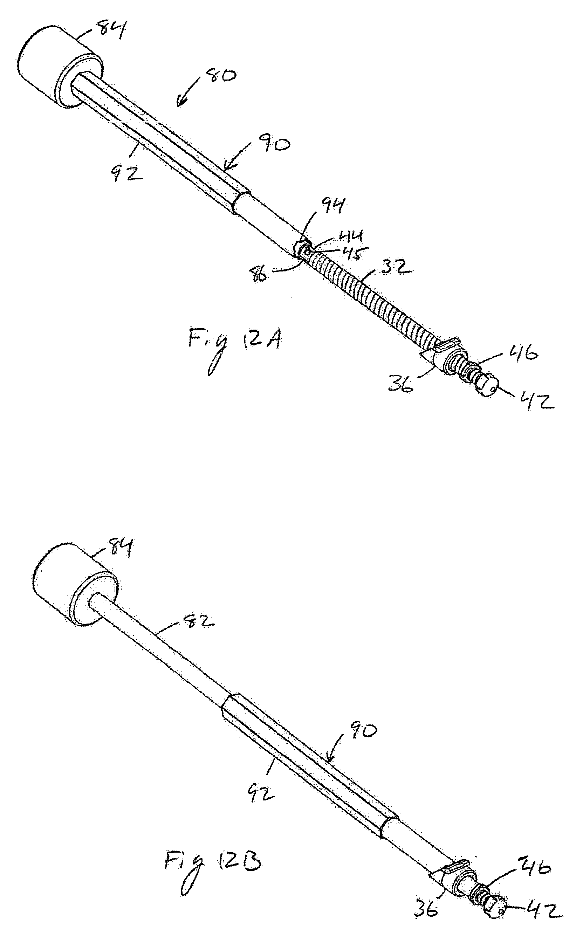

Once the reconstruction system has been assembled ex vivo, it is ready for insertion into the patient's knee as a completed unit. The surgeon has previously bored a tunnel 72 through the femur 70 and tibia 71 bones in the patient's knee, as illustrated in Fig. 10. Such a tunnel may be constructed by use of various conventional surgical drills, which can be introduced from the tibial end of the bone tunnel or the femoral end of the bone tunnel. Conventional ACL procedures utilize a bone tunnel having a 10 mm diameter.

A guide pin 74 is inserted through the bone tunnel 72, with an eyelet 76 thereof protruding from the tibia portion 72b of the bone tunnel 72. Each of the sutures 78/79 is looped (threaded) through the eyelet 76 and one of the guide holes 27 of hook member 18. The guide pin 74 is then used to pull (draw) the ends of the sutures 78 through the bone tunnel 72 and out the femoral bone tunnel portion 72. The surgeon then pulls on both ends of one of the sutures (e.g. suture 78), which pivots and/or maintains the hook member 18 in its insertion position, and which pulls the reconstruction system 1 through the bone tunnel 72 until the hook member 18 exits the femur portion 72a of the bone tunnel 72. Then, the surgeon pulls on both ends of the other suture (e.g. suture 79) to rotate (pivot) the hook member 18 into its anchor position, so that the tabs 26 engage the femur bone portions adjacent the bone tunnel 72 to anchor saddle member 16 against the femoral cortex. The surgeon can pull on the first suture (e.g. suture 78) to correct for any over-rotation of the hook member 18 caused by over-pulling of the other suture (e.g. suture 79). The sutures are later removed by pulling on just one end of each suture.

It should be noted that sutures 78/79 need not be threaded through the guide holes 27 exactly in the manner shown in Fig. 10. For example, both sutures 78/79 can be threaded through the same guide hole and still provide for the rotation of the hook member 18 in both directions. Specifically, one suture (e.g. suture 78) can be threaded through one of the guide holes 27, and the second suture (e.g. suture 79) can be threaded through a hole in the saddle member, then through the same guide hole 27, then back through the hole in the saddle. The hole in the saddle can be a specially provided hole, or could be an existing hole such as opening 20. Pulling the second suture 79 pulls the end of hook member 18 having the one guide hole 27 down toward the hole in saddle member 16, thus rotating (pivoting) hook member 18 into its insertion (folded) position for tunnel insertion. Pulling the first suture 78 rotates (pivots) the hook member 18 into its anchor position (extending orthogonally to bone tunnel 72). Thus, the unused guide hole could be eliminated from hook member 18.

Once the saddle member is anchored, nut 38 is tightened until tibia engagement projection 58 engages with the tibia bone portion adjacent the bone tunnel 72, and the proper tension is achieved on the graft inside bone tunnel 72. At this point, the knee can be cycled through its range of motion repeatedly, and then the tension on the graft readjusted intra-operatively if necessary using nut 38 until the desired finished graft tension is achieved. This system may further allow for tension re-adjustment for a short period of time post-operatively. Any portion of the bolt 32 and/or tab 44 extending beyond the bone anchor member 36 after system implementation can be cut to eliminate any protruding portions thereof.

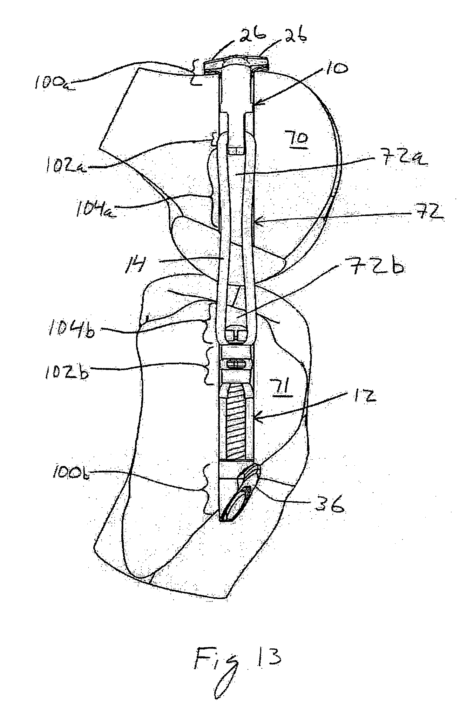

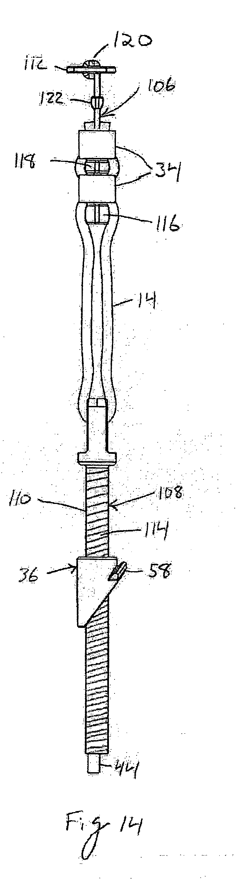

It is generally preferable that while nut 38 is adjusted (i.e. tightened or loosened against bone anchor member 36), that bolt 32 is prevented from rotating about its own longitudinal axis in order to prevent graft 14 from twisting. Thus, tab 44 at the end of bolt 32 can be used to hold the bolt 32 in place while nut 38 is adjusted. Figs. 11A and 11B illustrate an adjustment tool 80 that can be used to conveniently rotate nut 38 without rotating bolt 32. Adjustment tool 80 includes a shaft 82 with a handle 84 on one end and an engagement tab 86 with pin 88 on the other end. A sleeve 90 is slidably disposed over shaft 82, and has a gripping portion 92 at one end and engagement teeth 94 at the other end. An 0-ring 96 can be included between shaft 82 and sleeve 90 for stability. Figs. 12A and 12B illustrate the use of adjustment tool 80 (with graft 14 omitted for clarity), where shaft 82 is connected to bolt 32 by inserting pin 88 into hole 45 of tab 44 (as illustrated in Fig. 12A). Then, sleeve 90 is slid forward until teeth 94 engage with the external tabs 63 of nut 38, as illustrated in Fig. 12B. At this point, the surgeon pulls on bolt 32 to provide the desired graft tension, and rotates nut 38 about bolt 32 (by rotating sleeve 90) while preventing bolt 32 itself from rotating (by holding onto handle 84). The surgeon can feel or measure the graft tension externally, or the adjustment tool 80 can include a load cell (not shown) that measures the pulling force being exerted on the bolt (where rotating the nut will transfer that force from the adjustment tool to the tibial assembly).

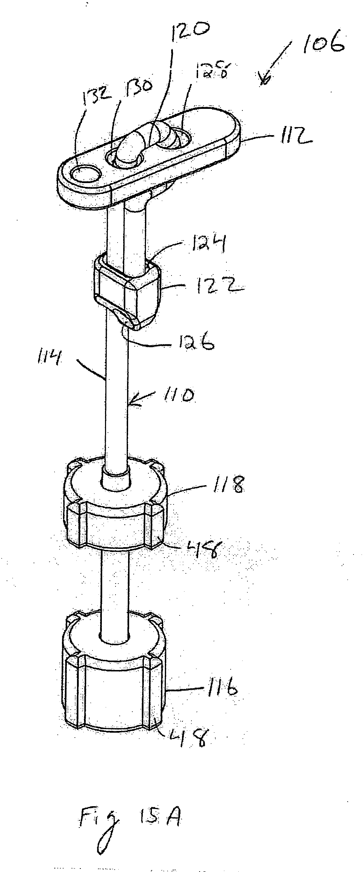

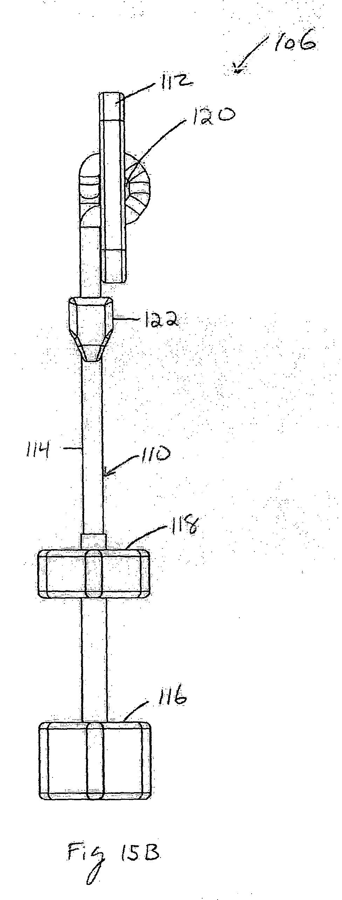

Figure 13 illustrates the reconstruction system 1 fully implemented inside the patient's knee. The system is anchored to the femur 70 via the hook members tabs 26, and to the tibia 71 via tibial engagement projection 58, at the ends of bone tunnel 72. The graft 14 is fixated to the femoral assembly 10 via tissue fixation surface 20a of saddle member opening 20, and fixated to the tibial assembly 12 via fixation ring 34, flange 46 and bolt 32. Both graft fixations are located inside bone tunnel 72.

Two healing zones 104a/104b are created by the reconstruction system of the present invention. A healing zone is where the graft 14 is placed in contact with the walls of the bone tunnel with sufficient pressure to promote the healing of the graft to the bone, and to ultimately result in a strong biological construct. One healing zone 104a is created just beyond the saddle member tissue fixation surface 20a (inside the femoral portion 72a of bone tunnel 72), and the other healing zone 104b is created just beyond the fixation ring 34 (inside the tibial portion 72b of bone tunnel 72). In each healing zone, the graft is gently pressed against the bone tunnel walls (e.g. by saddle member tissue presentation surface 20b for positioning graft 14 against the femoral bone, and by bolt head 42 which creates a raised presentation surface for positioning graft 14 against the tibia bone). The healing zones are dimensioned to exert sufficient forces between the graft and bone for forming a strong biological bond, yet not excessive forces sufficient to cause bone erosion, necrosis, and subsequent loss of fixation. The size of bolt head 42 can be varied to produce the desired presentation surface size, and could even be hollow and contain materials conducive to graft healing. Likewise, if flange 46 is fixed to the bolt 32 in an adjustable manner, the location of flange 46 along bolt 32 can be adjusted to optimize the location of tissue fixation (fixation zone) and the location of the adjacent healing zone.

As is evident from Figure 13, the two bone anchoring zones 100a/100b are located at the ends of bone tunnel 72, and are positioned to utilize the relatively hard cortical bone portions of the femur and tibia. The two graft fixation zones 102a/102b are located inside the bone tunnel 72, and involve graft 14 looping through saddle member 16, or ring-to-bolt graft fixation. The two graft healing zones 104a/104b, where the graft eventually forms attachments to the bone that will support the knee tendon, are located not only inside the bone tunnel 72, but interior to the graft fixation zones 102a/102b as well. Thus, the graft is healing with the softer cancellous inner portions of the femoral and tibial bones, and is less effected by any necrosis of the graft that will occur at the graft fixation zones 102a/102b. This separation of bone anchor, graft fixation and graft healing zones maximizes healing and minimizes graft failure. Graft portions in the graft fixation zones tend to necrose, and thus should be separated from the graft healing zones (for better healing) and from the bone anchoring zones (for more reliable anchoring). The mechanical fixation provided by the bone anchoring and graft fixation of the reconstruction system 1 secures the graft in place until biological fixation occurs in the healing zones that eventually replace the mechanical fixation. Once biological fixation is complete (e.g. around 12 weeks), the components of the femoral and tibial assemblies 10/12 preferably dissolve. Ideally, the reconstruction system 1 of the present invention will hold over 500 Newtons of tension on the graft 14 immediately after installation, which will allow the patient more mobility just after the ACL reconstruction surgery and during the 12 weeks of standard recovery.

Due to the fact that each component of anchoring, graft fixation, and graft healing require unique parameters for optimal benefit, the system of the present invention allows for independent control of each of these components. This independent control creates significant flexibility within the system, and eliminates conflicting forces that otherwise exist when such components are not independent or even performed concurrently (e.g., graft anchoring and fixation performed by interference screw at one bone type, etc.).

The present invention provides a complete, ex-vivo system solution, which is designed for ease of assembly and installation by the surgeon, for maximizing optimal surgical results, and for minimizing risk of surgical error. The completed reconstruction system can be installed as a single unit within a pre-formed tunnel of the femur and tibia, which simplifies installation and minimizes risk of error. It also allows for graft tension adjustment after graft fixation and bone anchoring, and even after the knee is cycled through its range of motion. Performance is enhanced because the system avoids any graft fixation directly to bone. Ex-vivo assembly of reconstruction system 1 and the use of fixation ring 34 ensures that all of graft 14 is properly fixated and equally tensioned, despite any non-standard or varying graft sizes. Lastly, saddle member 16 (with hook member 18) and bone anchor member 36 (with engagement projection 58) can reliably anchor the reconstruction system 1 to a wide variety of non-standard anatomical shapes and sizes.

### First Alternate Embodiment

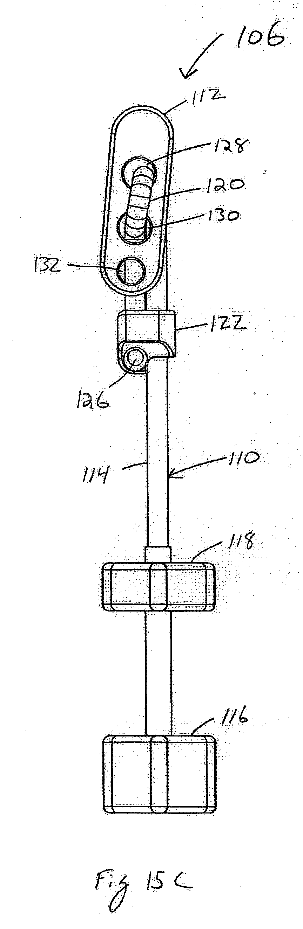

Figs. 14, 15A-15D, 16, 17A-17B and 18 illustrate a first alternate embodiment of the reconstruction system of the present invention, where the fixation of the graft using an opening for graft looping is on the tibial assembly, and the fixation of the graft using the compression ring is on the femoral assembly. This embodiment includes a femoral assembly 106 and a tibial assembly 108, with a graft 14 spanning therebetween, as best shown in Fig. 14.

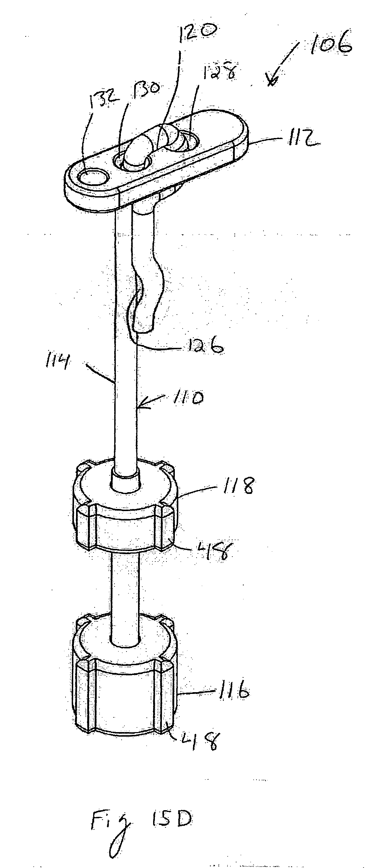

Femur assembly 106 is best shown in Figs. 15A-15D, and includes a bolt 110 and an anchor plate 112 (bone anchor) rotatably (pivotally) connected thereto. Bolt 110 includes a shaft 114, with a bolt head 116 and a flange 118 at one end thereof (which correspond to the bolt head 42 and flange 46 described above). The other end of the shaft 114 terminates in an open (e.g. hook shaped) or a closed (e.g. a ring shaped) loop 120. The portion of shaft 114 forming loop 120 is preferably, but not necessarily, integrally formed with the rest of shaft 114, where the end of shaft 114 bends back toward the mid-portion of shaft 114. The loop is preferably, but not necessarily, closed (e.g. by integrally forming a closed ring at the end of shaft 114, or by affixing the shaft's end to a mid portion of shaft 114 by using a clamp 122 as shown in Figs. 15A-15C or by welding as shown in Fig. 15D) for better structural strength. Alternately, a separate loop-shaped member can be attached to or formed on shaft 114, whereby shaft 114 is formed of two or more parts connected together. Clamp 122 preferably has a first (clamping) aperture(s) for fixing the end of shaft 114 to its mid-portion (e.g. via crimping, press-fitting, etc.), and a second (suture) aperture 126 through which a suture can be looped or threaded (as described later). In the case of Fig. 15D, aperture 126 is formed along a mid portion of the shaft (between a rounded portion of the shaft's end and the shaft's mid-portion, preferably with weld points on each side of aperture 126), where shaft 114 is shaped to form a second loop. Anchor plate 112 includes first and second holes 128/130 preferably formed in a center portion of plate 112, and a third hole 132 preferably formed near one end of plate 112. The portion of shaft 114 forming loop 120 extends up through hole 130 and down through hole 128, so that anchor plate 112 is rotatably (pivotally) attached to bolt 110 between an insertion position (as illustrated in Figs. 15B and 15C) and an anchor position (as illustrated in Figs. 15A and 15D).

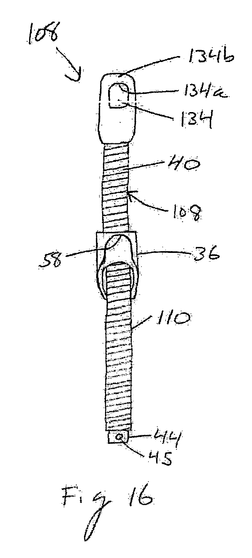

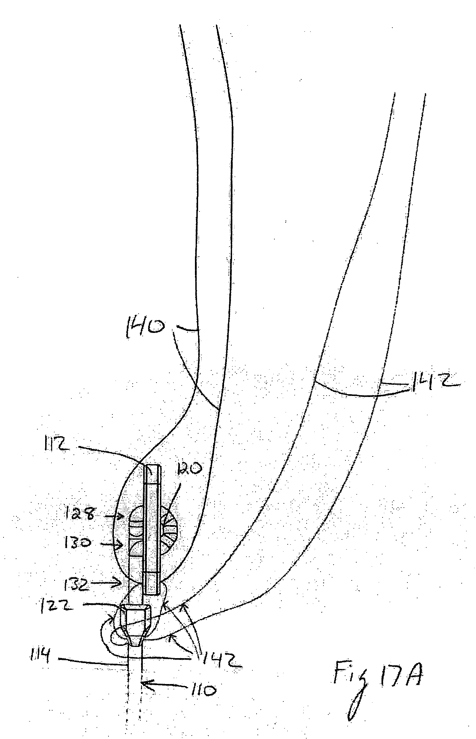

Tibial assembly 108 is essentially the same as the tibial assembly 12 described above, except that instead of terminating in the bolt head 42 and flange 46, bolt 110 terminates with an opening 134 through which graft 14 can be looped (threaded). Opening 134 includes a tissue fixation surface 134a defined in opening 134, and a tissue presentation surface 134b defined laterally and above opening 134, as illustrated in Fig. 16 (which correspond to the opening 20, tissue fixation surface 20a, and tissue presentation surface 20b, respectively, described above and shown in Fig. 2).

The components of the femoral and tibial assemblies 106/108 may be made of any of the materials listed above. One preferred combination of materials may include: any appropriate biocompatible material(s) such as stainless steel, titanium, nickel-titanium alloys, etc, for the bolt 110, clamp 122, and anchor plate 112; the 70%/30% PLA/poly DL-lactide biodegradable composition mentioned above for the graft fixation ring 34; and the 82%/18% PLA/ PLGA biodegradable composition mention above for the remaining components of the femoral and tibial assemblies 106/108.

Assembly of the first alternate embodiment of the reconstruction system is performed ex-vivo in essentially the manner as that described above, with only minor modification as noted below. Namely, once the bone tunnel 72 is measured, the graft 14 is looped through opening 134 of bolt 110, and the graft loose ends are inserted through the (expanded) fixation ring 34 and placed over bolt head 116 and bolt flange 118 so reconstruction system 1 has the proper overall length. The graft fixation ring 34 is then slipped over the bolt head 116 and bolt shaft 114 until apertures 50 of ring 34 engage with flange 118. Fixation ring 34 is then compressed, excite compressed, or wrapped around graft 14 to secure it to bolt 110 and flange 118, as described above. Bone anchor member 36 is slid onto bolt shaft 40, and nut 38 is threaded onto shaft 40 until it is positioned to engage with shoulder 56 and prevents bone anchor member 36 from sliding past a desired bone tunnel insertion position along bolt shaft 40. The resulting assembled system is shown in Fig. 14.

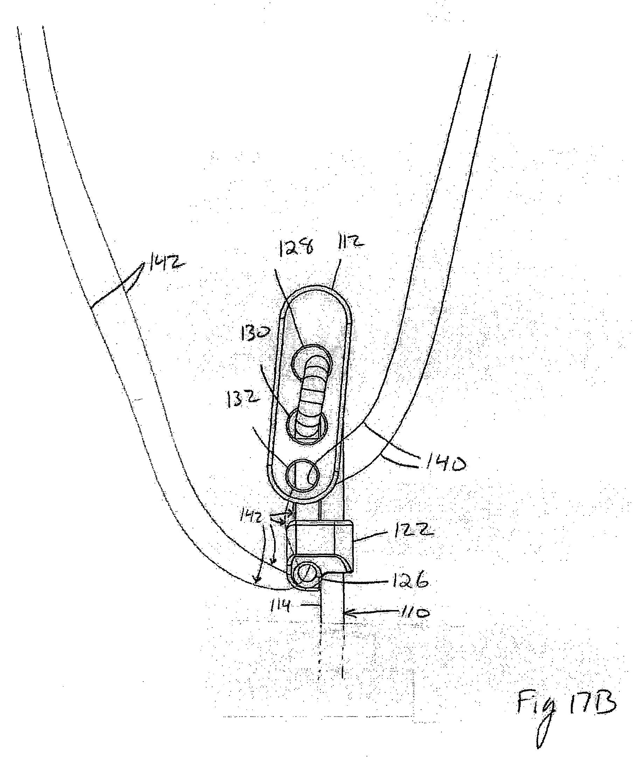

The implementation of the assembled reconstruction system of Fig. 14 into the bone tunnel 72 is similar to that explained above (with respect to Figs. 10, 11A-B, and 12A-B), but with some specific exceptions as noted below. First and second sutures 140/142 are attached to the femoral assembly as shown in Figs. 17A and 17B. Specifically, first suture 140 is threaded through the third hole 132 of anchor plate 112. Second suture 142 is threaded through the hole 126 of clamp 122, then through the third hole 132 of anchor plate 112, and then again through the hole 126 of clamp 122.

Once the ends of first and second sutures 140/142 are pulled through the bone tunnel 72 using the guide pin 74 (see above, and Fig. 10), the surgeon pulls on the second suture to draw the assembled reconstruction system through bone tunnel 72. Pulling on the second suture 142 also has the simultaneous affect of pulling the end of anchor plate 112 having third hole 132 down toward shaft 114 and clamp 122, thus rotating (pivoting) anchor plate into its insertion (folded) position, whereby anchor plate 112 will fit through bone tunnel 72. Once anchor plate 112 clears the upper end of femoral tunnel portion 72a, the surgeon pulls on the first suture 140, which rotates (pivots) the anchor plate 112 into its anchor position (extending laterally from bolt shaft 114). Thereafter, anchor plate 112 engages the femur bone portions (femoral cortex) adjacent the bone tunnel 72, thus anchoring the femoral assembly 106 in place. After the sutures are removed, nut 38 is tightened (preferably using adjustment tool 80) until tibia engagement projection 58 engages with the tibia bone portion (tibial cortex) adjacent the bone tunnel 72, and the proper tension is achieved on the graft 14 inside bone tunnel 72. At this point, the knee can be cycled through its range of motion repeatedly, and then the tension on the graft readjusted intra-operatively if necessary using nut 38 until the desired finished graft tension is achieved. This system may further allow for tension re-adjustment for a short period of time post-operatively. Any portion of the bolt 32 and/or tab 44 extending beyond the bone anchor member 36 after system implementation can be cut to eliminate any protruding portions thereof.

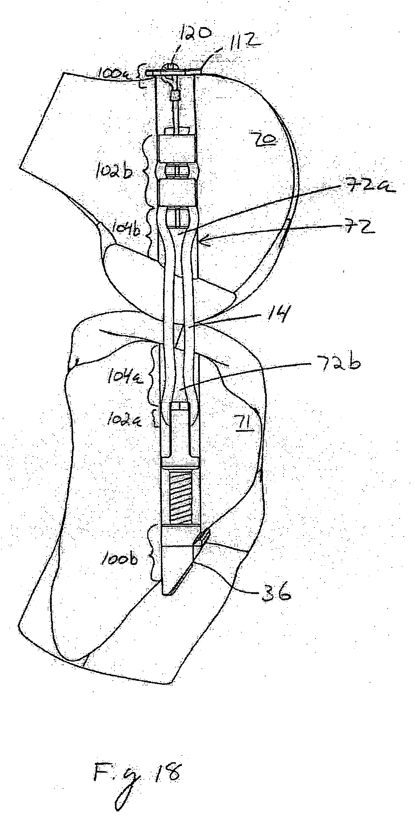

Fig. 18 illustrates the first alternate embodiment of the reconstruction system 1 fully implemented inside the patient's knee. The system is anchored to the femur 70 via the anchor plate 112, and to the tibia 71 via tibial engagement projection 58, at the ends of bone tunnel 72. The graft 14 is fixated to the femoral assembly 106 via fixation ring 34, flange 118 and bolt 110, and fixated to the tibial assembly 108 via graft fixation surface 134a of opening 134. Both graft fixations are located in graft fixation zones 102a/102b inside bone tunnel 72. Graft healing zones 104a/104b are created just beyond opening 134 and just beyond fixation ring 34, and enhanced by presentation surface 134a and bolt head 116, respectively. Bone anchoring zones 100a/100b are located at the ends of bone tunnel 72 by anchor plate 112 and by anchor member 36, and are positioned to utilize the relatively hard cortical bone portions of the femur and tibia. As illustrated in Fig. 18, the graft healing zones 104a/104b are located between the graft fixations zones 102a/102b, which in turn are located between the bone anchoring zones 100a/100b, for independently maximizing bone anchoring, graft fixation, and graft healing.

It is to be understood that the present invention is not limited to the embodiment(s) described above and illustrated herein, but encompasses any and all variations falling within the scope of the appended claims. For example, the term bolt as used herein can be any elongated rigid member (e.g. bolt, bar, beam, rod, pin, post, wire, etc.) capable of transferring a load. Materials, processes and numerical examples described above are exemplary only, and should not be deemed to limit the claims. Further, as is apparent from the claims and specification, not all method steps necessarily need be performed in the exact order illustrated or claimed (e.g. ring 34 could be inserted on bolt shaft 40 from the end thereof opposite from bolt head 42). Hook member 18 and cap member 30 could be integrally formed together. Guide holes 27 could be formed vertically through tabs 26, instead of horizontally as shown in the figures. Tabs 26 could be formed and deployable separately, instead of as an integrally formed member. The reconstruction system 1 could instead be inserted from the femoral side of bone tunnel 72, and/or inserted with femoral assembly anchoring to the tibia, and visa versa. The reconstruction system 1, or portions thereof, can be used in a bone tunnel having only one open end (e.g. a bone tunnel formed only partially through the bone), as opposed to two open ends shown in the figures. Graft fixation ring 34 could have any number of apertures 50, including none. Graft fixation ring 34 also need not have the circular shape shown in the drawings (e.g. could be square or irregularly shaped for enhanced fixation). Graft fixation ring 34 could be two or more separate rings (although multiple rings may be harder to position).

While threads are the ideal means for adjusting the length of the reconstruction system by providing convenient and continuous length adjustment in both directions, other means of incrementally adjustable attachment of bone anchor member 36 to bolt 32 could be used (e.g. ratchet teeth, locking channels, etc.). While shafts 40, 82, and 114 are shown as having round cross-sections, such shafts can have any regular or irregular shape. An inflatable heating bladder could be used instead of heating coil 66 to heat compress fixation ring 34. Needles or other rigid members could be used instead of sutures to push or pull the reconstruction system it through the bone tunnel. While Figs. 17A/17B show suture 142 looped through aperture 126 of clamp 122, any other means for slidably securing suture to clamp 122 or bolt 110 can be used instead, such as a hole, channel or notch formed directly in shaft 114. Hook member 18 (with tabs 26 thereon) and anchor plate 112 need not necessarily be rotatably connected to saddle member 16 or loop 120. Rather, tabs 26 or plate 112 need only be movably attached thereto (so that their profile increases after insertion through the bone tunnel), which includes rotation, flexing, translation, deformation and/or expansion of these bone anchor elements relative to the rigid member on which they are mounted (where the use of sutures to move the hook member 18 or anchor plate 112 may not be necessary).

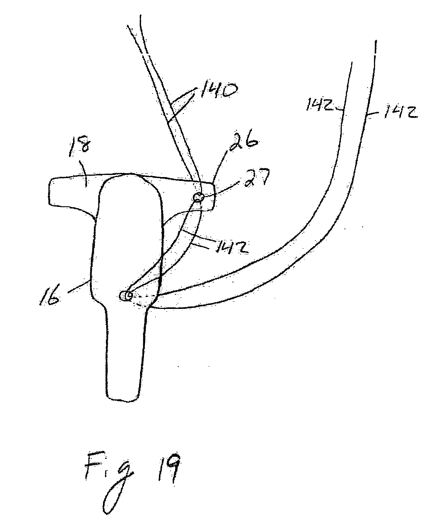

Features of the embodiments of Figs. 13 and 18 can be combined and/or swapped. For example, shaft 114 of Fig. 18 could terminate in an opening similar to opening 20, so that loop 120 and anchor plate 112 of the first alternate embodiment could be used in the embodiment of Fig. 13. Likewise, saddle member 16 could terminate in a shaft having a head and a flange (for graft fixation ring 34), so that hook member 18 could be used conjunction with the first alternate embodiment of Fig. 18. In fact, both the femoral and tibial assemblies could employ graft fixation ring members for graft fixation. While openings 20/134 are shown as closed eyelets for structure integrity and to minimize any potential snag points, these openings need not necessarily be completely closed. The threading of the sutures of femoral assembly 106 as shown in Figs. 17A-17B can be employed for the femoral assembly 10 using a single guide hole 27 and a suture hole 150 formed in the saddle member 16, as shown in Fig. 19.

Lastly, the femoral assembly 10 or 106 could be used separately by itself, without tibial assembly 12 or 108, and vice versa, as well as in modified form or in conjunction with other well known bone anchoring devices that anchor to bone portions (i.e. bone material) adjacent a bone tunnel (e.g. cortex bone portions, the bone tunnel sidewalls, etc.), even for tissue anchoring applications not involving a femur, a tibia, and/or an anterior cruciate ligament. For example, if graft 14 is a "bone-tendon" graft (meaning the graft includes a bone attached at one end, such as a graft harvested from the Achilles bone), then the bone can be anchored to the bone tunnel using a pin or screw, and the free end of the graft can be anchored to the bone tunnel using the adjustable tibial assembly 12 or 108. If graft 14 is a "bone-tendon-bone" graft (meaning the graft includes bones attached at both ends, such as a hamstring graft), then a pin or screw can be used to anchor one bone to the bone tunnel, and the other bone can be adjustably fixed to the bolt 32 or 110 (e.g. by passing the bolt shaft through a hole in the bone, by using a cage-like member to capture the bone and adjustably affix it to the bolt, by using sutures to adjustably affix the bone to the bolt, etc.).

## Claims

1. A reconstruction system for inserting a graft into a pre-bored bone tunnel, comprising:
a first bone anchor for anchoring to bone material adjacent the bone tunnel;
a shaft connected to the first bone anchor; and
a ring member extending around the shaft and the graft and exerting a fixation force on the graft toward the shaft to secure the graft to the shaft;
wherein the ring member is one of a ring compressed around the graft and the shaft, an elongated member wrapped around the graft and the shaft, and a ring excite compressed around the shaft and the graft.

2. The system of claim 1, wherein the shaft includes a presentation surface that is adjustably connected to the shaft for adjusting a distance between the presentation surface and the first anchor.

3. The system of claim 1, wherein the shaft includes a flange that is adjustably connected to the shaft for adjusting a distance between the flange and the first anchor.

4. The system of claim 1, wherein the shaft includes a flange and a raised presentation surface which are both integrally formed as a single unitary unit, and wherein the single unitary unit is adjustably connected to the shaft for adjusting a distance between the single unitary unit and the first anchor.

5. The system of claim 1, wherein the shaft includes a flange and the ring member is excite compressed over the flange.

6. The system of claim 5, wherein the ring member includes first and second portions for securing the graft to the shaft, and wherein the flange is disposed between the first and second ring member portions.

7. The system of claim 6, wherein the ring member includes at least one aperture engaged with the flange.

8. The system of claim 1, further comprising:
a rigid member connected to a second bone anchor for anchoring to bone material adjacent the bone tunnel, wherein the rigid member includes an opening through which the graft is looped, and wherein the second bone anchor is movably connected to the rigid member between a folded position, and an unfolded position where the second bone anchor extends generally laterally from the rigid member;
the rigid member further includes a slot and a pin extending across the slot, the second bone anchor being rotatably connected to the pin;
the second bone anchor further comprises a pair of tabs extending from the central portion, wherein each of the tabs includes a suture hole formed therein; and
a pair of sutures each threaded through one of the suture holes.

9. The system of claim 1, further comprising:
a rigid member connected to a second bone anchor for anchoring to bone material adjacent the bone tunnel, wherein the rigid member includes an opening through which the graft is looped, and wherein the second bone anchor is movably connected to the rigid member between a folded position, and an unfolded position where the second bone anchor extends generally laterally from the rigid member;
the rigid member further includes a slot and a pin extending across the slot, the second bone anchor being rotatably connected to the pin;
a first suture threaded through a hole formed in the second bone anchor; and
a second suture threaded through a suture hole formed through the rigid member, then through the hole formed in the second bone anchor, and then back through the suture hole.

10. The system of claim 1, further comprising:
a rigid member connected to a second bone anchor for anchoring to bone material adjacent the bone tunnel, wherein the rigid member includes an opening through which the graft is looped, and wherein the second bone anchor is movably connected to the rigid member between a folded position, and an unfolded position where the second bone anchor extends generally laterally from the rigid member;
one end of the rigid member terminates in a loop; and
the second bone anchor is an elongated member having first and second holes through which the loop traverses to form the rotatable connection therebetween.

11. The system of claim 10, wherein the loop is integrally formed with the rigid member.

12. The system of claim 10, further comprising:
a clamp member for securing the rigid member one end to a mid portion of the rigid member in a manner such that the loop is closed.

13. The system of claim 12, wherein the clamp member includes a hole through which a suture can be passed.

14. The system of claim 10, wherein the elongated member includes a third hole through which a suture can be passed.

15. The system of claim 14, further comprising:
a first suture threaded through the third hole; and
a second suture threaded through a suture hole disposed along a mid portion of the rigid member, then through the third hole, and then back through the suture hole.

16. The system of claim 1, wherein:
the first bone anchor is movably connected to the shaft between a folded position, and an unfolded position where the first bone anchor extends generally laterally from the shaft;
one end of the shaft terminates in a loop; and
the first bone anchor is an elongated member having first and second holes through which the loop traverses to form the rotatable connection therebetween.

17. The system of claim 16, wherein the loop is integrally formed with the shaft.

18. The system of claim 16, further comprising:
a clamp member for securing the shaft one end to a mid portion of the shaft in a manner such that the loop is closed.

19. The system of claim 18, wherein the clamp member includes a hole through which a suture can be passed.

20. The system of claim 16, wherein the elongated member includes a third hole through which a suture can be passed.

21. The system of claim 20, further comprising:
a first suture threaded through the third hole; and
a second suture threaded through a suture hole disposed along a mid portion of the shaft, then through the third hole, and then back through the suture hole.

22. The system of claim 1, wherein:
the first bone anchor is movably connected to the shaft between a folded position, and an unfolded position where the first bone anchor extends generally laterally from the shaft;
the shaft further includes a slot and a pin extending across the slot, and the first bone anchor is rotatably connected to the pin;
the first bone anchor further comprises a pair of tabs extending from the central portion;
a suture hole is formed in each of the tabs;
the system further comprises a pair of sutures each threaded through one of the suture holes.

23. The system of claim 1, further comprising:
the first bone anchor is movably connected to the shaft between a folded position, and an unfolded position where the first bone anchor extends generally laterally from the shaft;
the shaft further includes a slot and a pin extending across the slot, and the first bone anchor is rotatably connected to the pin;
a first suture threaded through a hole formed in the first bone anchor; and
a second suture threaded through a suture hole formed through the rigid member, then through the hole formed in the first bone anchor, and then back through the suture hole.

24. The system of claim 1, further comprising:
the first bone anchor is movably connected to the shaft between a folded position, and an unfolded position where the first bone anchor extends generally laterally from the shaft;
a rigid member connected to a second bone anchor for anchoring to bone material adjacent the bone tunnel, wherein the rigid member includes an opening through which the graft is looped.

25. The system of claim 24, wherein the rigid member includes a second shaft, and wherein the second bone anchor is slidably attached to the second shaft in an adjustable manner.

26. The system of claim 25, wherein the second shaft includes a threaded portion, and the second bone anchor includes a nut having internal threads that engage with the second shaft threaded portion.

27. The system of claim 25, wherein the second bone anchor comprises:
a cylindrical body; and
a bone engagement projection extending from the cylindrical body.

28. The system of claim 27, wherein:
the cylindrical body includes first and second sidewall portions;
the first sidewall portion is shorter than the second sidewall portion; and
the bone engagement projection extends from the first sidewall portion.

29. The system of claim 28, wherein the bone engagement projection does not extend from the second sidewall portion.

30. A method of assembling a graft reconstruction system, comprising:
threading a graft through an opening disposed on a first shaft, wherein a first bone anchor is connected to the first shaft, and
fixing a ring member around the graft and a second shaft such that the ring member exerts a fixation force on the graft toward the shaft to secure the graft to the second shaft,
wherein the second shaft is connected to a second bone anchor;
wherein the fixing of the ring member includes compressing the ring member around the graft and a shaft, or wrapping an elongated member around the graft and the shaft.

31. The method of claim 30, wherein the fixing of the ring member includes the compressing of the ring member around the graft and the shaft while the ring member is in an excited state.

32. The method of claim 31, further comprising:
expanding the ring member while the ring member is in an excited state before the compressing of the ring member.

33. The method of claim 30, further comprising:
attaching a pair of sutures to one of the first or second anchors.

34. The method of claim 30, wherein the shaft includes a presentation surface adjustably connected to the shaft, and wherein the method further comprises:
adjusting a position of the presentation surface along the shaft for adjusting a distance between the presentation surface and the first anchor.

35. The method of claim 30, wherein the shaft includes a flange adjustably connected to the shaft, and wherein the method further comprises:
adjusting a position of the flange along the shaft for adjusting a distance between the flange and the first anchor.

36. The method of claim 30, wherein the shaft includes a flange and a raised presentation surface which are both integrally formed as a single unitary unit that is adjustably connected to the shaft, and wherein the method further comprises:
adjusting a position of the single unitary unit along the shaft for adjusting a distance between the single unitary unit and the first anchor.

37. The method of claim 30, wherein the shaft includes a flange, and wherein the fixing of the ring member includes the compressing of the ring member around the graft and the shaft and over the flange.

38. The method of claim 30, wherein the ring member includes first and second portions for securing the graft against the shaft, and wherein the flange is disposed between the first and second ring member portions.

39. The method of claim 38, wherein the ring member includes at least one aperture engaged with the flange.

40. The method of claim 38, further comprising:
placing a tension on the graft before the fixing of the ring member.

41. The method of claim 30, wherein the first bone anchor is slidably connected to the shaft in an adjustable manner, and wherein the method further includes:
adjusting a position of the first bone anchor along the shaft.

42. The method of claim 41, wherein the adjusting of the first bone anchor position includes rotating a nut having internal threads that engage a threaded portion of the shaft.

43. The method of claim 42, wherein the adjusting of the first bone anchor position further includes grasping the shaft to prevent rotation of the shaft as the nut is rotated.

44. The method of claim 41, wherein the first bone anchor comprises a cylindrical body having first and second sidewall portions and a bone engagement projection extending from the cylindrical body, and wherein the first sidewall portion is shorter than the second sidewall portion and the bone engagement projection extends from the first sidewall portion.

45. The method of claim 30, wherein:
the first bone anchor is movably connected to the first shaft between a folded position, and an unfolded position where the first bone anchor extends generally laterally from the first shaft.

46. The method of claim 45, wherein one end of the first shaft terminates in a loop, and the first bone anchor is an elongated member having tirst and second holes through which the loop traverses to form the rotatable connection therebetween, the first bone anchor further including a third hole, and a suture hole is disposed along a mid portion of the first shaft, the method further comprising:
threading a first suture through the third hole; and
threading a second suture through the suture hole, then through the third hole, and then back through the suture hole.

47. The method of claim 46, wherein the first shaft includes a clamp member for securing the first shaft one end to the mid portion of the first shaft in a manner such that the loop is closed, and wherein the suture hole is formed in the clamp member.

48. The method of claim 45, wherein the first bone anchor includes a central portion rotatably connected to the first shaft, and first and second tabs extending from the central portion, the method further comprising:
connecting a first suture to the first tab; and
connecting a second suture to the second tab.

49. The method of claim 48, wherein the connecting of the first suture includes threading the first suture through a suture hole formed in the first tab, and wherein the connecting of the second suture includes threading the second suture through a suture hole formed in the second tab.

50. The method of claim 45, wherein the first bone anchor includes a central portion rotatably connected to the first shaft, and first and second tabs extending from the central portion, and wherein one of the tabs includes a first hole and the first shaft includes a second hole, the method further comprising:
threading a first suture through the first hole; and
threading a second suture through the second hole, then through the first hole, and then back through the second hole.

51. The method of claim 30, wherein:
the second bone anchor is movably connected to the second shaft between a folded position, and an unfolded position where the second bone anchor extends generally laterally from the second shaft.

52. The method of claim 51, wherein one end of the second shaft terminates in a loop, and the second bone anchor is an elongated member having first and second holes through which the loop traverses to form the rotatable connection therebetween, the second bone anchor further including a third hole, and a suture hole is disposed along a mid portion of the second shaft, the method further comprising:
threading a first suture through the third hole; and
threading a second suture through the suture hole, then through the third hole, and then back through the suture hole.

53. The method of claim 52, wherein the second shaft includes a clamp member for securing the second shaft one end to the mid portion of the second shaft in a manner such that the loop is closed, and wherein the suture hole is formed in the clamp member.

54. The method of claim 51, wherein the second bone anchor includes a central portion rotatably connected to the second shaft, and first and second tabs extending from the central portion, the method further comprising:
connecting a first suture to the first tab; and
connecting a second suture to the second tab.

55. The method of claim 54, wherein the connecting of the first suture includes threading the first suture through a suture hole formed in the first tab, and wherein the connecting of the second suture includes threading the second suture through a suture hole formed in the second tab.

56. The method of claim 51, wherein the second bone anchor includes a central portion rotatably connected to the second shaft, and first and second tabs extending from the central portion, and wherein one of the tabs includes a first hole and the second shaft includes a second hole, the method further comprising:
threading a first suture through the first hole; and
threading a second suture through the second hole, then through the first hole, and then back through the second hole.

57. The method of claim 31, wherein the compressing of the ring member includes:
wrapping a flexible band around the ring member, wherein the band defines a compression aperture in which the ring member is located;
manipulating the band to shrink a size of the compression aperture; and
heating the band;
wherein the band exerts an inwardly compressive force on the ring member while heating the ring member.

58. The method of claim 57, wherein the inwardly compressive force and the heat are applied to the ring in a generally uniform manner by the band.
